# EUROPEAN PATENT SPECIFICATION

(11) **EP 3 466 450 B1**
(45) Date of publication and mention of the grant of the patent: **19.07.2023**
(21) Application number: 16903909.6
(22) Date of filing: 31.05.2016
(51) Int. Cl.: A61L 2/08, B65B 55/04, B65B 55/08

(54) **ELECTRON BEAM STERILIZATION DEVICE AND ELECTRON BEAM STERILIZATION METHOD**
ELEKTRONENSTRAHLBESTRAHLUNGSVORRICHTUNG UND ELEKTRONENSTRAHLBESTRAHLUNGSVERFAHREN
DISPOSITIF DE STÉRILISATION PAR FAISCEAU D'ÉLECTRONS ET PROCÉDÉ DE STÉRILISATION PAR FAISCEAU D'ÉLECTRONS

(43) Date of publication of application: 10.04.2019
(73) Proprietor: Mitsubishi Heavy Industries Machinery Systems, Ltd., Hyogo-ku, Kobe-shi Hyogo 652-8585 (JP)
(72) Inventor: MIZUKAWA, Kenji, Nagoya-shi Aichi 453-0862 (JP); UEDA, Atsushi, Nagoya-shi Aichi 453-0862 (JP); TSUO, Atsushi, Nagoya-shi Aichi 453-0862 (JP)
(74) Representative: Hoffmann Eitle
(86) International application number: PCT/JP2016/002621
(87) International publication number: WO 2017/208278

(56) References cited:
- JP-A- 2002 104 333
- JP-A- 2002 171 949
- JP-A- 2006 314 407
- JP-A- 2013 242 218
- JP-A- 2016 097 971
- US-A1- 2015 108 366

## Description

### Technical Field

The present invention relates to an electron beam sterilization device and an electron beam sterilization method that are suitable for sterilizing containers to be filled with foods, beverages, medical products and the like, and other sterilization objects, with an electron beam.

### Background Art

Before a container is filled with filling substances such as foods, beverages and medical products, the container or a preformed body for the container, which is called a preform, is sterilized.

For the sterilization of the container or the preform, chemicals such as peracetic acid or hydrogen peroxide or ultraviolet irradiation have been used. In recent years, there has been considered a sterilization technique by irradiation with an electron beam, which has a higher sterilizing power than ultraviolet light.

For surely performing the sterilization by the electron beam irradiation, it is necessary to uniformly irradiate the whole of the container with the electron beam. However, when the irradiation with the electron beam is performed while the container is being conveyed in a state of being grasped by a gripper or a holder which is called a gripper, the irradiation of the holding portion with the electron beam results in an imperfect state. In the case where the container is a plastic bottle, typically, a neck part (or a bottleneck part) to which a cap is attached is held by the gripper, and therefore, the dose for sites hidden by the gripper results in an insufficient state, or some sites are not irradiated with the electron beam.

Hence, Patent Literature 1 proposes compensating for the insufficiency of the dose for the sites hidden by the gripper by providing, in the gripper, a passage through which the electron beam passes.

Further, in Patent Literature 2, a first external irradiation device (42) irradiates a half side surface of the preform with the electron beam, and a second external irradiation device (43) irradiates the other half side surface of the preform with the electron beam. When the preform moves from an area for the electron beam irradiation by the first external irradiation device to an area for the electron beam irradiation by the second external irradiation device, a transfer of the preform by grippers is performed.
A further related prior art may be found in Patent Literature 3, which discloses an electron beam irradiation device.

### Citation List

### Patent Literature

Patent Literature 1: JP 2013-133111 A (FIG. 3)
Patent Literature 2: JP 2015-030516 A (FIG. 2, FIG. 3)
Patent Literature 3: US 2015/0108366 A1

### Summary of Invention

### Technical Problem

According to the proposal of Patent Literature 1, it is possible to supplement the dose of the electron beam for a range that is irradiated with the electron beam having passed through the passage, but the other site is not irradiated with the electron beam.

Further, in the proposal of Patent Literature 2, the preform is conveyed using a rotating body called a star wheel, and therefore, the preform is gripped by the gripper, at both ends in the circumferential direction of the rotating body, with respect to both the gripper on the transferring side and the gripper on the transferred side. Accordingly, there is a concern that the electron beam does not reach the grip portion of the gripper even if the sterilization process of the preform is performed for each half side surface.

If the container that is an object of the sterilization with the electron beam is rotated about the axis of the gripper, it is possible to eliminate the sites hidden by the gripper. However, since a mechanism for rotating the container is necessary, this makes the cost of the device rise and requires a larger occupied space for the device.

Hence, the present invention has an object to provide an electron beam sterilization device capable of eliminating areas that are not irradiated with the electron beam due to the gripper, without providing the mechanism for rotating the sterilization object.

### Solution to Problem

The electron beam sterilization device of the present invention is defined by claim 1. The method of the present invention is defined by claim 7.

The dependent claims are related to optional features and particular embodiments.

An electron beam sterilization device in the present invention includes: a first gripper configured to receive a sterilization object and then convey the sterilization object in a conveyance direction to a grasp switching position while gripping the sterilization object; a second gripper configured to receive the sterilization object from the first gripper and then convey the sterilization object downstream while gripping the sterilization object; and an electron beam irradiation unit configured to irradiate the sterilization object that passes through an irradiation area, with an electron beam, the irradiation area containing the grasp switching position and forward and rearward positions of the grasp switching position.

According to the present invention, even sites that are not irradiated with the electron beam before grasp switching because of being gripped by the gripper can be irradiated with the electron beam after the grasp switching, and therefore, the sites that are not irradiated with the electron beam are not produced.

In the present invention, each of the first gripper and the second gripper is configured to move along a linear conveyance path while gripping a predetermined number of the sterilization objects without rotating the sterilization objects about the axis of the grippers.

By sterilizing the sterilization objects by batch processing and conveying the sterilization objects along the linear conveyance path in this way, it is possible to simplify the configuration of the device, compared to the case of using a rotary conveyance device with use of a star wheel. Further, according to the present invention, it is only necessary to perform the irradiation with the electron beam while the preform gripped by the gripper is being conveyed along the linear conveyance path, and therefore, it is not necessary to provide a mechanism for rotating the preform about the axis.

Further, in the present invention, it is preferable that each of the first gripper and the second gripper grip a predetermined number of the sterilization objects that are arrayed on a line, and one or both of the first gripper and the second gripper relatively come close to each other in a direction perpendicular to a direction in which a plurality of the sterilization objects are arrayed, so that grasp switching of the sterilization objects from the first gripper to the second gripper is performed at the grasp switching position.

If the grasp switching of the sterilization object arrayed on a line is performed by the first gripper and the second gripper in this way, it is possible to facilitate the configuration of the first gripper and the second gripper for the grasp switching, compared to the case where the sterilization objects are arrayed on a plurality of lines.

In the present invention, it is preferable that the first gripper and the second gripper grip the sterilization object on different positions from each other respectively.

Thereby, even the sites that are not irradiated with the electron beam before grasp switching because of being gripped by the gripper can be irradiated with the electron beam after the grasp switching, and therefore, sites that are not irradiated with the electron beam before and after the grasp switching are not produced.

In the present invention, it is preferable that the irradiation area for the electron beam in the electron beam irradiation unit be put under a reduced-pressure atmosphere.

The electron beam scatters and attenuates in air, and therefore, there is a concern that the electron beam cannot reach a necessary site. Hence, by putting the irradiation area under the reduced-pressure atmosphere, the electron beam is made to reach, for example, a bottom of the container, and the effect of the sterilization is sufficiently obtained.

In the case where the irradiation area is put under the reduced-pressure atmosphere, the electron beam irradiation unit may include: a pre-irradiation chamber containing a receiving position at which the first gripper receives a predetermined number of the sterilization objects; an irradiation chamber in which the sterilization objects conveyed by the first gripper are irradiated with the electron beam while grasp switching of the sterilization objects to the second gripper is being performed; a post-irradiation chamber to which the sterilization objects irradiated with the electron beam in the irradiation chamber are conveyed by the second gripper; and an electron beam generation device configured to perform irradiation with the electron beam in the irradiation chamber, and it is preferable that the irradiation chamber have a higher vacuum degree than the pre-irradiation chamber and the post-irradiation chamber.

The second gripper may increase a pitch between the sterilization objects gripped, while the sterilization objects are being conveyed from the irradiation chamber to the post-irradiation chamber or after the sterilization objects are conveyed to the post-irradiation chamber.

For example, in the case where the sterilization objects are preforms, the preforms are molded into containers having a larger size than the preforms, at a place to which the preforms are conveyed from the post-irradiation chamber, and in accordance with the size of the containers, the pitch between positions for gripping the sterilization objects is increased at the time when the conveyance has been performed.

In the present invention, the sterilization object may be a container including an opening part and a storage space for holding a content, the storage space communicating with the opening part, or a preformed body for the container. The sterilization object may be gripped by the first gripper and the second gripper, in an upright state in which the opening part is oriented upward in a vertical direction or in an inverted state that is upside-down. In this case, when the electronic beam irradiation unit includes: a first electron beam generation device configured to irradiate the sterilization object with the electron beam from an upper side to a lower side in a vertical direction; and a second electron beam generation device configured to irradiate the sterilization object with the electron beam from the lower side to the upper side in the vertical direction, and thereby, it is possible to uniformly irradiate the outer circumferential surface and inner circumferential surface of the container or the preformed body for the container, with the electron beam.

Further, in the case where the sterilization object is a container or a preformed body for the container, the first gripper can grip the sterilization object by gripping three spots around the opening part, and the second gripper can grip the sterilization object by supporting three spots around the opening part that do not interfere with the first gripper.

When each of the first gripper and the second gripper, so to speak, has a three-point support structure in this way, it is possible to reduce sites which hide the sterilization object. Although it is difficult to apply such a three-point support structure to the gripper of the rotary conveyance device with use of a star wheel, it is possible to easily apply the three-point support structure to the gripper that moves along the linear conveyance path.

The present invention provides also an electron beam sterilization method including: a step in which a first gripper receives a sterilization object and then conveys the sterilization object in a conveyance direction to a grasp switching position while gripping the sterilization object; a step in which a second gripper receives the sterilization object from the first gripper and then conveys the sterilization object downstream while gripping the sterilization object; and an electron beam irradiation step of irradiating the sterilization object that passes through an irradiation area, with an electron beam, the irradiation area containing the grasp switching position and forward and rearward positions of the grasp switching position, wherein each of the first gripper and the second gripper moves along a linear conveyance path while gripping a predetermined number of the sterilization objects without rotating the sterilization objects about the axis of the grippers.

In the electron beam sterilization method in the present invention, it is possible to apply the above-described preferable means in the electron beam sterilization device described above.

### Advantageous Effects of Invention

According to the present invention, even sites that were not irradiated with the electron beam before grasp switching can be irradiated with the electron beam after the grasp switching, and therefore, the sites that are not irradiated with the electron beam are not produced. Moreover, it is only necessary to perform the irradiation with the electron beam while the preform gripped by the gripper is being conveyed along the linear conveyance path, and therefore, it is not necessary to provide a mechanism for rotating the preform about the axis.

### Brief Description of Drawings

FIG. 1A shows a blow-molding device according to the present embodiment and is a plan view showing the schematic configuration, and FIG. 1B shows the blow-molding device and is a plan view showing a conveyance path for preforms and containers, corresponding to FIG. 1A.
FIG. 2A shows a main configuration of a sterilization unit of the blow-molding device in FIGs. 1A and 1B and is a diagram viewed from a conveyance direction F, and FIG. 2B shows the main configuration of the sterilization unit and is a diagram viewed from a width direction W.
FIG. 3A, FIG. 3B and FIG. 3C are diagrams showing operations of the blow-molding device in FIGs. 1A and 1B.
FIG. 4A, FIG. 4B, FIG. 4C and FIG. 4D are diagrams showing operations of the blow-molding device following FIG. 3C.
FIG. 5A, FIG. 5B, FIG. 5C and FIG. 5D are diagrams showing operations of the blow-molding device following FIG. 4D.
FIG. 6A, FIG. 6B, FIG. 6C and FIG. 6D are diagrams showing operations of the blow-molding device following FIG. 5D.
FIG. 7A shows one type of preferable grippers in the present embodiment which is a lower grip type gripper, and FIG. 7B shows another type of preferable grippers which is an upper grip type gripper; and
FIG. 8A, FIG. 8B and FIG. 8C are diagrams showing the process of grasp switching of a preform PF by the grippers in FIG. 7A and FIG. 7B.

### Description of Embodiments

Hereinafter, an example in which an electron beam sterilization device and electron beam sterilization method according to the present invention are applied to a blow-molding device 1 that molds, by blow molding, test tube-shaped preforms PF consecutively conveyed from upstream into containers B made of plastic will be described.

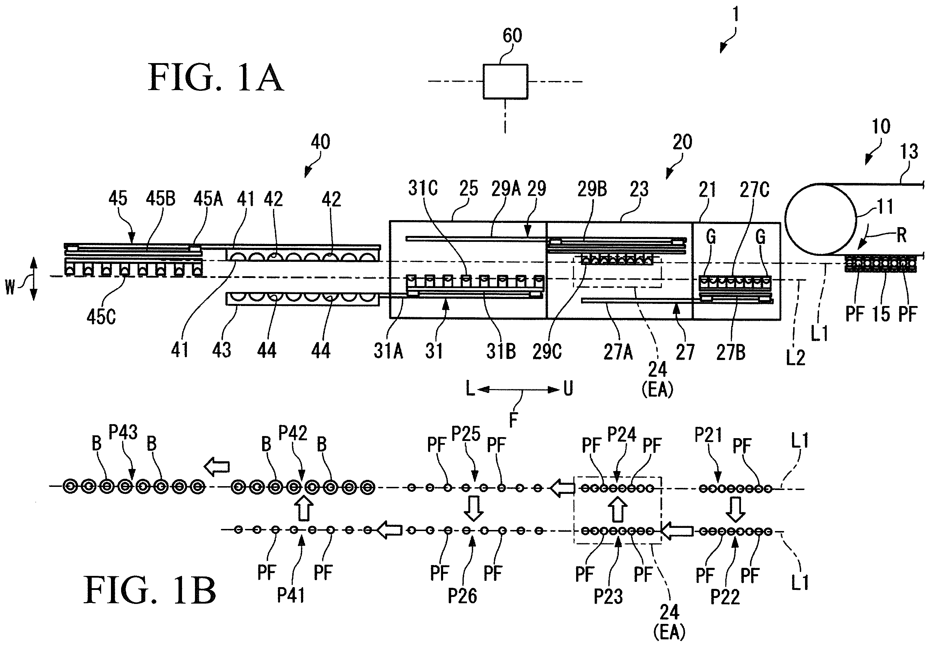

As shown in FIG. 1A and FIG. 1B, the blow-molding device 1 includes a carry-in unit 10 that carries in the preforms PF heated to a predetermined temperature for blow molding, a sterilization unit 20 that sterilizes the preforms PF carried by the carry-in unit 10 by the irradiation with an electron beam, a molding unit 40 that molds the preforms PF sterilized in the sterilization unit 20 into the containers B, and a control unit 60 that controls the operations of the carry-in unit 10, the sterilization unit 20 and the molding unit 40.

The blow-molding device 1 has a characteristic in that the blow-molding device 1 can completely irradiate the whole of the inner circumferential surface and outer circumferential surface of the preform PF with the electron beam in the sterilization unit 20. Each configuration of the blow-molding device 1 will be described below.

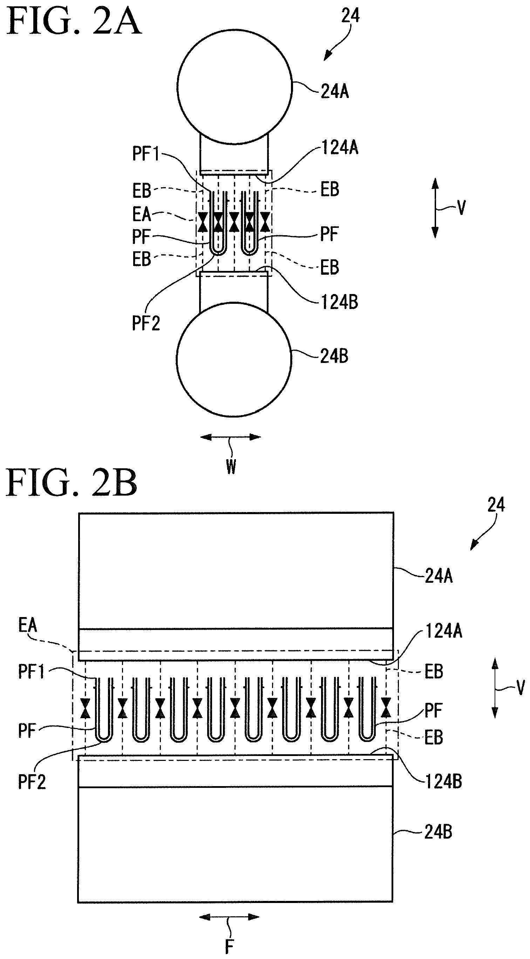

In FIG. 1A and FIG. 1B, the preform PF and the container B are conveyed along a conveyance direction F, and in the present embodiment, an upstream U and a downstream L are defined with reference to the conveyance direction F of the preform PF or the container B. Further, a direction perpendicular to the conveyance direction F is referred to as a width direction W. Furthermore, as shown in FIG. 2A and FIG. 2B, in the carry-in unit 10, the sterilization unit 20 and the molding unit 40, the preform PF is conveyed and processed in an upright state in which an opening part (PF1) is disposed on the upper side in the vertical direction. The same goes for the container B that is molded from the preform PF.

### [Carry-in Unit 10]

The carry-in unit 10 carries into the sterilization unit 20, the preforms PF heated to a temperature appropriate for the molding in the molding unit 40 by an unillustrated heating device.

As shown in FIG. 1A and FIG. 1B, the carry-in unit 10 includes a rotating body 11 that is rotated, for example, clockwise (R), a conveyance belt 13 as endless conveyance means in which one end is wound around the rotating body 11 and the other is wound around an unillustrated rotating body, and a conveyance means 15 that receives the preforms PF conveyed to a predetermined position by the conveyance belt 13 and conveys the preforms PF to the sterilization unit 20.

By rotating the rotating body 11, the heated preforms PF are conveyed by the conveyance belt 13. The conveyance means 15 waits at a predetermined position on a conveyance path of the conveyance belt 13, and at the predetermined position, receives a predetermined number of preforms PF from the conveyance belt 13. After receiving the preforms PF, the conveyance means 15 conveys the predetermined number of preforms PF to the sterilization unit 20 while gripping the preforms PF. Thus, the conveyance means 15 includes means (grippers) for gripping the preforms PF and a conveyance means for conveying the grippers. The conveyance means (the conveyance means 15) linearly reciprocates between a position at which the conveyance means receives the preforms PF from the conveyance belt 13 and a position at which the conveyance means transfers the preforms PF to the sterilization unit 20. A path along which the conveyance means 15 reciprocates and an extended line thereof are referred to as a first path L1.

### [Sterilization Unit 20]

The sterilization unit 20 irradiates the conveyed preforms PF with the electron beam under an atmosphere in which the pressure is reduced compared to the atmospheric pressure, and thereby, sterilizes the inner circumferential surface and the outer circumferential surface.

As shown in FIG. 1A and FIG. 1B, the sterilization unit 20 includes a pre-irradiation chamber 21 in which the preforms PF are carried, an irradiation chamber 23 that is adjacent to the pre-irradiation chamber 21 on the downstream L side and in which the carried preforms PF are irradiated with the electron beam, and a post-irradiation chamber 25 in which the preforms PF irradiated with the electron beam in the irradiation chamber 23 is conveyed.

Between the pre-irradiation chamber 21, the irradiation chamber 23 and the post-irradiation chamber 25, unillustrated opening-closing doors through which the preforms PF pass are provided respectively.

In the pre-irradiation chamber 21, the irradiation chamber 23 and the post-irradiation chamber 25, the respective pressures are reduced by an unillustrated vacuum pump, and thereby, are maintained at desired vacuum degrees. When the sterilization unit 20 sterilizes the preforms PF, the vacuum degree of the irradiation chamber 23 is controlled to become higher, and the vacuum degrees of the pre-irradiation chamber 21 and the post-irradiation chamber 25 are controlled to become lower than that of the irradiation chamber 23. Here, the sterilization unit 20 includes the three chambers of the pre-irradiation chamber 21, the irradiation chamber 23 and the post-irradiation chamber 25, in order to shorten the time required to reduce the pressure of the irradiation chamber 23 to the desired vacuum degree. That is, by providing the pre-irradiation chamber 21 on the upstream U side of the irradiation chamber 23 and setting it to a certain vacuum degree, it is possible to keep the vacuum degree of the irradiation chamber 23 to at least the vacuum degree of the pre-irradiation chamber 21, even if the opening-closing door between the pre-irradiation chamber 21 and the irradiation chamber 23 is opened. Thereby, it is possible to shorten the time required to reduce the pressure to the desired vacuum degree, compared to the case of carrying in the preforms PF while the irradiation chamber 23 is open to the atmosphere and then reducing the pressure from the atmospheric pressure to the desired pressure without providing the pre-irradiation chamber 21. The same goes for the irradiation chamber 23 and the post-irradiation chamber 25. It should be understood that performing the irradiation with the electron beam under the reduced-pressure atmosphere and providing the pre-irradiation chamber 21, the irradiation chamber 23 and the post-irradiation chamber 25 are preferable embodiments in the present invention, but are not elements that limit the present invention.

As shown in FIG. 1A and FIG. 1B, the sterilization unit 20 includes a first conveyance means 27, a second conveyance means 29 and a third conveyance means 31 for conveying the preforms PF.

The first conveyance means 27 is provided from the pre-irradiation chamber 21 to the irradiation chamber 23. As shown in FIG. 1A and FIG. 1B, the first conveyance means 27 receives the preforms PF from the conveyance means 15 of the carry-in unit 10 at a position P21 in the interior portion of the pre-irradiation chamber 21, and conveys the preforms PF to a position P23 in the interior portion of the irradiation chamber 23. The position P21, the position P23 and the like are shown in FIG. 1B.

As shown in FIG. 1A and FIG. 1B, the first conveyance means 27 includes a linear guide rail 27A that is laid from the pre-irradiation chamber 21 to the irradiation chamber 23, a running carriage 27B that reciprocates between a position P22 and the position P23 along the guide rail 27A, that is, along the conveyance direction F, and a gripper group 27C that reciprocates together with the running carriage 27B by being mounted on the running carriage 27B. The preforms PF gripped by the gripper group 27C are conveyed along a second path L2.

In the gripper group 27C corresponding to the first gripper in the present invention, grippers G provided correspondingly to the number of the preforms PF to be conveyed at one time are disposed so as to be arrayed in the conveyance direction F. As the gripper G in the present embodiment, an arbitrary form may be adopted as long as a part of the preform PF can be surely gripped.

The gripper group 27C can perform advance-retreat movement in the width direction W relative to the running carriage 27B. The advance-retreat means the movement from the second path L2 to the first path L1 and the movement from the first path L1 to the second path L2. The advance-retreat of the gripper group 27C is performed for receiving the preforms PF from the conveyance means 15 and for transferring the preforms PF to the second conveyance means 29 of the irradiation chamber 23. The advance-retreat operation may be performed such that a gripper group 29C advances or retreats and thereby comes close to the gripper group 27C, or may be performed such that both the gripper group 27C and the gripper group 29C advance or retreat and thereby come close to each other.

The plurality of preforms PF gripped by the gripper group 27C are arranged so as to be linearly arrayed on a line along the conveyance direction F, as shown in FIG. 1B, and the plurality of preforms PF, in each of which the opening part PF1 is gripped by the gripper G, are arranged so as to be arrayed at the same height in the vertical direction (V), as shown in FIG. 2A and FIG. 2B.

Next, as shown in FIG. 1A and FIG. 1B, the second conveyance means 29 is provided from the irradiation chamber 23 to the post-irradiation chamber 25. As shown in FIG. 1A and FIG. 1B, the second conveyance means 29 receives the preforms PF from the gripper group 27C of the first conveyance means 27 at the position P23 in the interior portion of the irradiation chamber 23, and conveys the preforms PF to a position P25 in the interior portion of the post-irradiation chamber 25. The position P23 corresponds to the grasp switching position in the present invention.

As shown in FIG. 1A, the second conveyance means 29 includes a linear guide rail 29A that is laid from the irradiation chamber 23 to the post-irradiation chamber 25, a running carriage 29B that reciprocates between a position P24 and the position P25 along the guide rail 29A, and the gripper group 29C that reciprocates together with the running carriage 29B by being mounted on the running carriage 29B. The preforms PF gripped by the gripper group 29C corresponding to the second gripper in the present invention is conveyed on the first path L1 along the conveyance direction F.

Next, as shown in FIG. 1A, the third conveyance means 31 is provided from the post-irradiation chamber 25 to the molding unit 40. As shown in FIG. 1A and FIG. 1B, the third conveyance means 31 receives the preforms PF from the gripper group 29C of the second conveyance means 29 at a position P26 in the interior portion of the post-irradiation chamber 25, and conveys the preforms PF to a position P41 in the molding unit 40.

As shown in FIG. 1A, the third conveyance means 31 includes a linear guide rail 31A that is laid from the post-irradiation chamber 25 to the molding unit 40, a running carriage 31B that reciprocates between the position P26 and the position P41 along the guide rail 31A, and a gripper group 31C that reciprocates together with the running carriage 31B by being mounted on the running carriage 31B. The preforms PF gripped by the gripper group 31C are conveyed on the second path L2 along the conveyance direction F.

The second conveyance means 29 and the third conveyance means 31 perform operations similar to those of the first conveyance means 27 although there are differences in the position where the preforms PF are conveyed, and therefore, the description below is omitted.

As shown in FIG. 1A, the sterilization unit 20 includes an electron beam generation device 24 in the interior portion of the irradiation chamber 23.

As shown in FIG. 2A and FIG. 2B, the electron beam generation device 24 includes an upper irradiation unit 24A that irradiates the preforms PF with an electron beam EB from the upper side of the preforms PF and a lower irradiation unit 24B that irradiates the preforms PF with the electron beam EB from the lower side of the preforms PF. The upper irradiation unit 24A is provided for the purpose of sterilizing the inner circumferential surface of the preform PF mainly, and the lower irradiation unit 24B is provided for the purpose of sterilizing the outer circumferential surface of the preform PF mainly. The pressure of the interior of the irradiation chamber 23 is reduced to a predetermined vacuum degree, such that the electron beam EB emitted from the upper irradiation unit 24A can enter the interior of the preform PF and can reach the bottom surface.

As shown in FIG. 2A and FIG. 2B, in the upper irradiation unit 24A, an emission surface 124A for the irradiation with the electron beam EB is oriented downward in the vertical direction V, and is parallel with a horizontal direction H. Further, in the lower irradiation unit 24B, an emission surface 124B for the irradiation with the electron beam EB is oriented upward in the vertical direction V, and is parallel with the horizontal direction H. FIG. 2A shows two preforms PF, and this shows that the preform PF moves in the width direction W from the left side to the right side in the figure.

The upper irradiation unit 24A and the lower irradiation unit 24B may have the same specification except the difference in the orientation of the irradiation with the electron beam EB, or may have different specifications, for example, such that the intensity of the electron beam EB emitted from the upper irradiation unit 24A is higher than that from the lower irradiation unit 24B.

With respect to an area (an electron beam irradiation area EA) for the irradiation with the electron beam EB by the upper irradiation unit 24A and the lower irradiation unit 24B, as shown in FIG. 2B, a length in the conveyance direction F of the preforms PF is set so as to be longer than a distance in which the preforms PF to be processed at one time, that is, to be batch-processed, are arranged. Accordingly, by conveying the preforms PF to be batch-processed to the electron beam irradiation area EA after positioning, it is possible to sterilize all of the preforms PF to be batch-processed, without moving the preforms PF or the upper irradiation unit 24A and lower irradiation unit 24B thereafter.

Further, as shown in FIG. 2A, a length of the electron beam irradiation area EA in the width direction W is set so as to be longer than a distance in which the preforms PF are transferred from the first conveyance means 27 to the second conveyance means 29.

Thus, the sterilization unit 20 can continuously irradiate the preforms PF to be processed as a batch with the electron beam EB, at the position of the grasp switching from the gripper group 27C to the gripper group 29C where the preforms PF are transferred from the first conveyance means 27 to the second conveyance means 29, and forward and rearward positions thereof.

Further, in the sterilization unit 20, for the preforms PF to be batch-processed, as shown in FIG. 2A and FIG. 2B, the distances from the emission surface 124A of the upper irradiation unit 24A to each of the sites are equal to each other, and the distances from the emission surface 124A of the lower irradiation unit 24B to each of the sites are equal to each other. What is the distance is constant means that the distances from the emission surface 124A or emission surface 124B to the same site of the respective preforms PF are equal to each other, for example, that the distances from the emission surface 124A to the respective opening parts PF1 of the plurality of preforms PF are equal to each other and the distances from the emission surface 124B to the respective bottom parts PF2 of the plurality of preforms PF are equal to each other.

Thus, in the sterilization unit 20, the respective sites of each of the preforms PF to be batch-processed are evenly irradiated with the electron beam EB from the electron beam generation device 24.

### [Molding Unit 40]

The molding unit 40 molds into the containers B, the preforms PF on which the sterilization process has been performed by the electron beam irradiation in the sterilization unit 20.

As shown in FIG. 1A, the molding unit 40 includes a pair of molding die 41 and molding die 43 into which the preforms PF heated to an adequate temperature necessary for molding are inserted. The molding unit 40 performs the blow of a blow-molding gas from an unillustrated supply source to the interior portion of the preforms PF held by the molding dies 41, 43, and thereby, performs the stretch blow molding for the containers B.

The molding dies 41, 43, respectively, include die elements 42, 44 provided correspondingly to the number of the preforms PF or containers B to be processed at one time. The molding dies 41, 43 perform such an advance-retreat operation that they come close to or depart from each other, and the stretch blow molding is performed in a vicinal state that they are close to each other.

As shown in FIG. 1A, the molding unit 40 includes a fourth conveyance means 45.

The fourth conveyance means 45 is provided from the molding unit 40 to a predetermined area in the downstream L of the molding unit 40. The fourth conveyance means 45 receives the molded containers B at the position P41, and conveys the containers B to a position P43 located on the downstream side of the molding unit 40.

The fourth conveyance means 45 includes a linear guide rail 45A that is laid from the molding unit 40 to the position P43 located on the downstream side of the molding unit 40, a running carriage 45B that reciprocates between the position P42 and the position P43 along the guide rail 45A, and a gripper group 45C that reciprocates together with the running carriage 45B by being mounted on the running carriage 45B.

### [Operation of Blow-Molding Device 1]

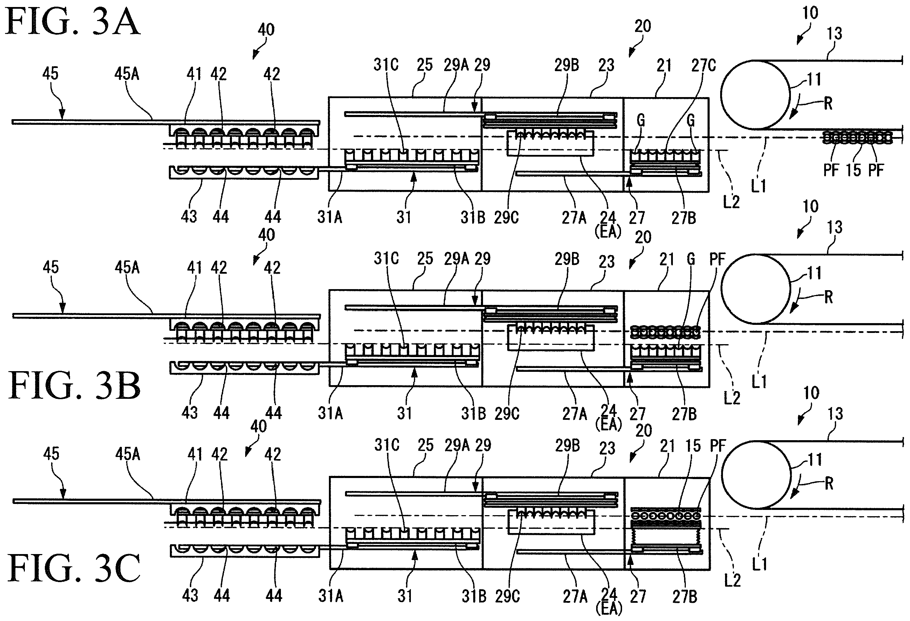

Next, the operation of the blow-molding device 1 will be described with reference to FIG. 3A to FIG. 6D.

The preforms PF (FIG. 3A) conveyed to a predetermined position by the conveyance belt 13 of the carry-in unit 10 are gripped by the conveyance means 15, and are conveyed to the pre-irradiation chamber 21 of the sterilization unit 20. The plurality of preforms PF is referred to as a first group. The conveyance means 15 conveys the preforms PF of the first group to the front of the running carriage 27B waiting at the position P21 (FIG. 3B). This conveyance is performed along the first path L1.

In the first conveyance means 27, the waiting gripper group 27C advances toward the conveyance means 15, and the gripper group 27C receives the preforms PF held by the conveyance means 15 (FIG. 3C).

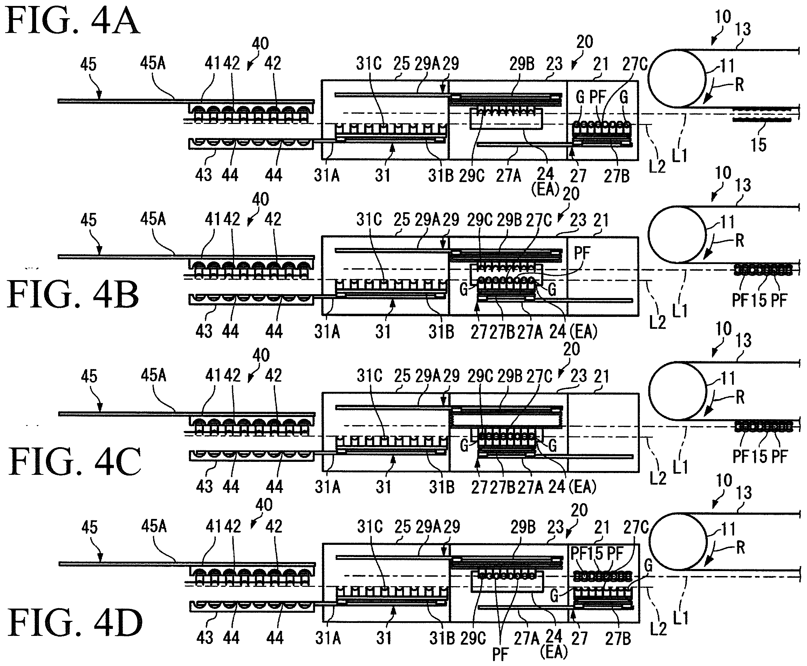

After the gripper group 27C receives the preforms PF, the first conveyance means 27 retreats to the position P22, which is the original position (FIG. 4A), and then, the running carriage 27B conveys the preforms PF of the first group to the position P23 in the irradiation chamber 23 (FIG. 4B). The position P23 is included in the electron beam irradiation area EA for the irradiation with the electron beam EB from the upper irradiation unit 24A and the lower irradiation unit 24B that constitute the electron beam generation device 24, and therefore, the sterilization process of the preforms PF conveyed to the position P23 have been already performed at this time.

When the preforms PF have moved to the position P23, the running carriage 29B of the second conveyance means 29 waiting at the position P24 advances toward the preforms PF placed on the second path L2, together with the gripper group 29C. Then, the second conveyance means 29 grips the preforms PF, by operating the gripper group 29C (FIG. 4C). After the grip of the gripper group 27C of the first conveyance means 27 is released, the second conveyance means 29 retreats to the original position P24 on the first path L1 while gripping the preforms PF by the gripper group 29C (FIG. 4D).

The position P24 is also in the electron beam irradiation area EA, and therefore, the preforms PF conveyed to the position P24 are irradiated with the electron beam over the period of the movement from the first path L1 to the second path L2, so that the sterilization process is performed.

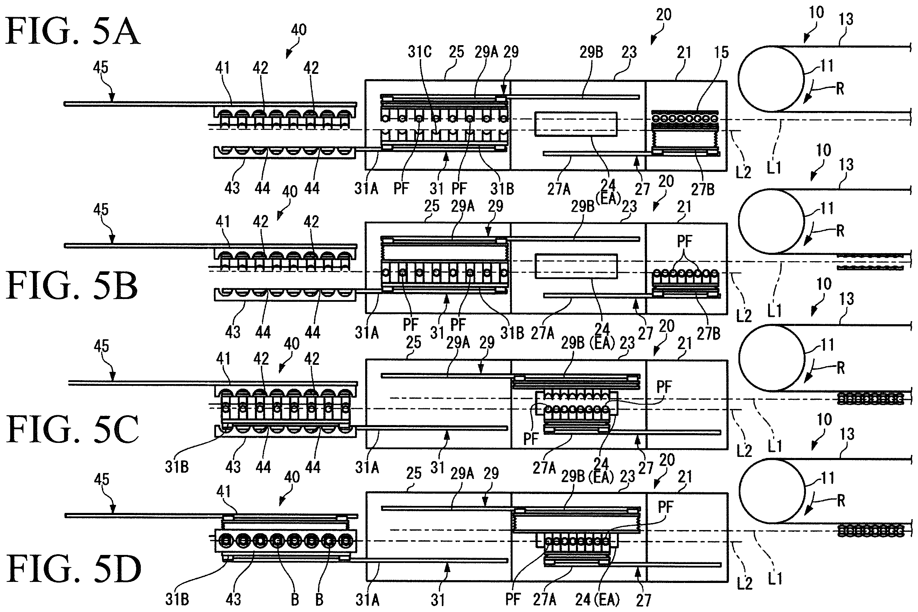

Next, the running carriage 29B of the second conveyance means 29 moves to the position P25 while gripping the preforms PF of the first group by the gripper group 29C (FIG. 5A). In the process of this movement, the interval between the grippers G of the gripper group 29C is increased. This is for the molding of the preforms PF into the containers B having a larger diameter in the molding unit 40. The increased interval is determined with reference to the diameter of the containers B, such that adjacent containers B do not interfere with each other.

After moving to the position P25, the running carriage 29B advances toward the running carriage 31B of the third conveyance means 31 waiting at the position P26, and transfers the preforms PF from the gripper group 29C to the gripper group 31C of the running carriage 31B (FIG. 5B) .

After the gripper group 31C receives the preforms PF, the running carriage 31B moves from the position P26 to the position P41 in the molding unit 40 (FIG. 5C). By this movement, the preforms PF go out of the post-irradiation chamber 25 of the sterilization unit 20, and are supplied for blow molding.

After the preforms PF are conveyed to the position P41 located between the molding die 41 and the molding die 43 in the molding unit 40, each of the molding die 41 and the molding die 43 advances toward the preforms PF placed on the first path L1, and holds the preforms PF in a predetermined state. Then, the blow molding is performed (FIG. 5D). At this time, the running carriage 45B of the fourth conveyance means 45 advances together with the molding die 41, and grips the preforms PF by the gripper group 45C and the gripper group 31C.

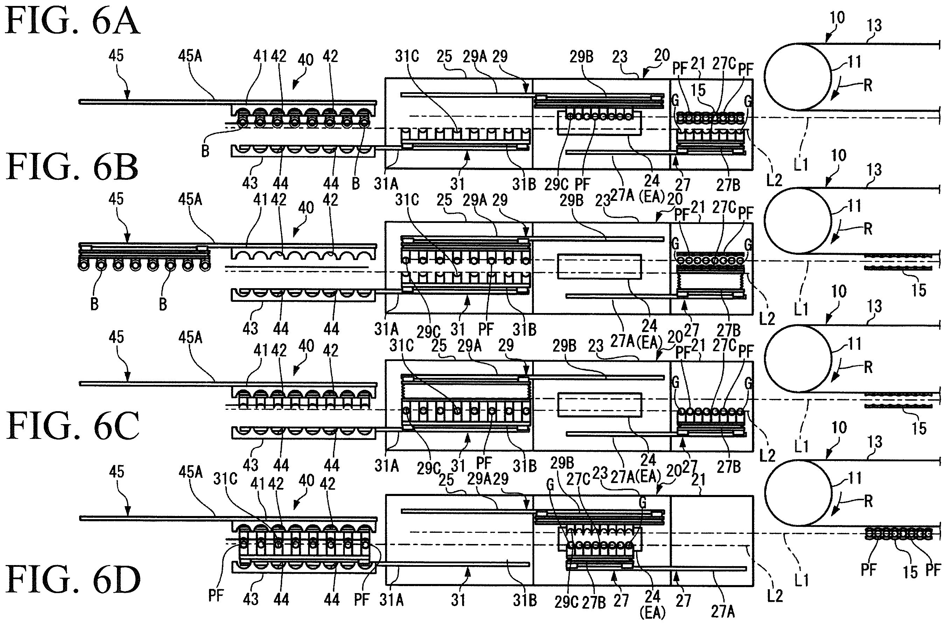

After the blow molding finishes, the molding die 41 and the molding die 43 return to the original positions, respectively (FIG. 6A).

After conveying the containers B to the position P43 on the downstream side, the running carriage 45B gripping the containers B transfers the containers B to an unillustrated conveyance means, and thereafter, reruns to the former position P42 of the molding die 41 (FIG. 6B and FIG. 6C). At this position, the running carriage 45B waits for the preforms PF of a subsequent second group to be conveyed to the position P41 (FIG. 6D).

### [Effect of Blow-Molding Device 1]

In the sterilization unit 20 of the blow-molding device 1, the preforms PF gripped by the gripper group 27C is placed in the electron beam irradiation area EA, during the grasp switching associated with the transfer to the gripper group 29C, and before and after the grasp switching.

Therefore, according to the present embodiment, even sites that were not irradiated with the electron beam before the grasp switching because of being gripped by the gripper group 27C can be irradiated with the electron beam after the grasp switching, and therefore, sites that are not irradiated with the electron beam are not produced in a period before and after the grasp switching. Moreover, in the embodiment, it is only necessary to irradiate, with the electron beam, the preforms PF linearly conveyed by the gripper group 27C and the gripper group 29C that are involved in the transfer, and therefore, it is not necessary to provide a mechanism for rotating the preforms PF about the axis.

Next, in the blow-molding device 1, each of the gripper group 27C and the gripper group 29C conveys the predetermined number of preforms PF along the linear conveyance path, while gripping the predetermined number of preforms PF, in the sterilization unit 20.

The sterilization unit 20 requires a shielding structure for preventing the electron beam and X-rays generated by the irradiation of the electron beam from leaking to the exterior. However, it is possible to simplify the shielding structure, compared to a rotary conveyance device with a star wheel.

Next, in the blow-molding device 1, the gripper group 27C and the gripper group 29C grip the predetermined number of preforms PF arrayed on a line, and relatively come close to each other in the direction perpendicular to the direction in which the plurality of preforms PF are arrayed on a line, and thereby, the grasp switching of the preforms PF from the gripper group 27C to the gripper group 29C is performed at the grasp switching position.

In the present embodiment, the batch type gripper group 27C and gripper group 29C linearly move in this way, and therefore, it is possible to simplify the conveyance mechanism, compared to the case of using the rotary conveyance device with a star wheel.

Next, in the sterilization unit 20, the irradiation chamber 23 containing the electron beam irradiation area EA is put under the reduced-pressure atmosphere.

Accordingly, particularly, the electron beam EB emitted from the upper irradiation unit 24A can enter the interior portion of the preform PF and can reach the inner circumferential surface of the bottom part PF2, and therefore, it is possible to completely sterilize the inner circumferential surface of the preform PF.

Next, in the sterilization unit 20, the gripper group 29C increases a grip pitch of the preforms PF, while conveying the preforms PF from the irradiation chamber 23 to the post-irradiation chamber 25.

Accordingly, it is not necessary to provide a space for increasing the grip pitch of the preforms PF after the preforms PF pass through the sterilization unit 20. Therefore, according to the present embodiment, it is possible to realize a space-saving blow-molding device 1.

The preform PF in the embodiment is gripped and conveyed by the gripper group 27C and the gripper group 29C, in the upright state in which the opening part PF1 is oriented upward in the vertical direction, and the sterilization unit 20 includes the upper irradiation unit 24A that irradiates the preforms PF with the electron beam EB from the upper side to the lower side in the vertical direction and the lower irradiation unit 24B that irradiates the preforms PF with the electron beam from the lower side to the upper side in the vertical direction.

Accordingly, it is possible to uniformly irradiate the outer circumferential surface and inner circumferential surface of the preform PF with the electron beam. Here, even if the process is performed in an inverted state in which the opening parts of the preforms PF are oriented downward, it is possible to exert the same effect.

The preferable embodiment of the present invention has been described above. Other than this, without departing from the spirit of the present invention, some of the configurations described in the above embodiment can be selected, or can be appropriately modified to different configurations.

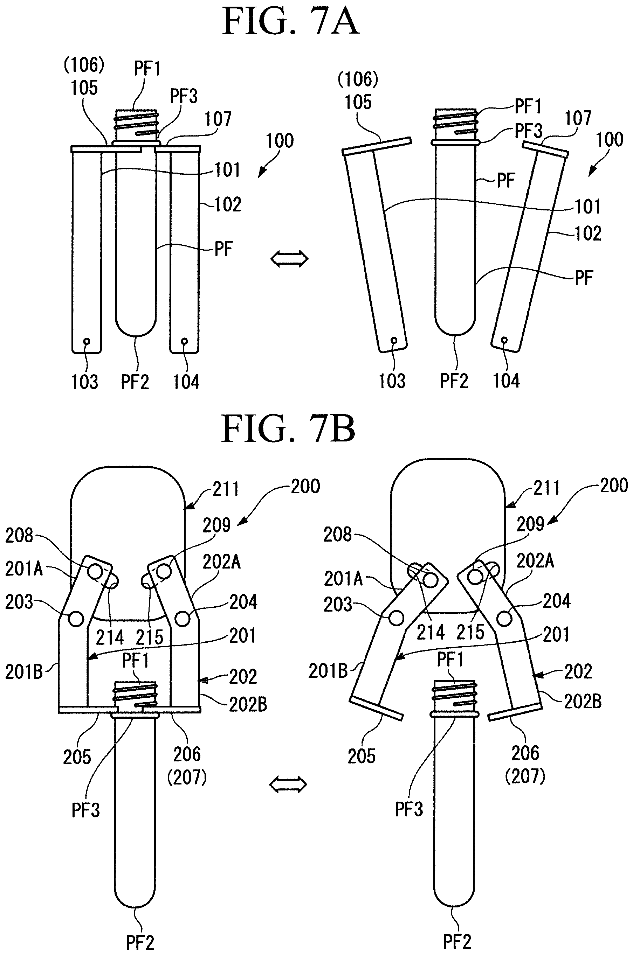

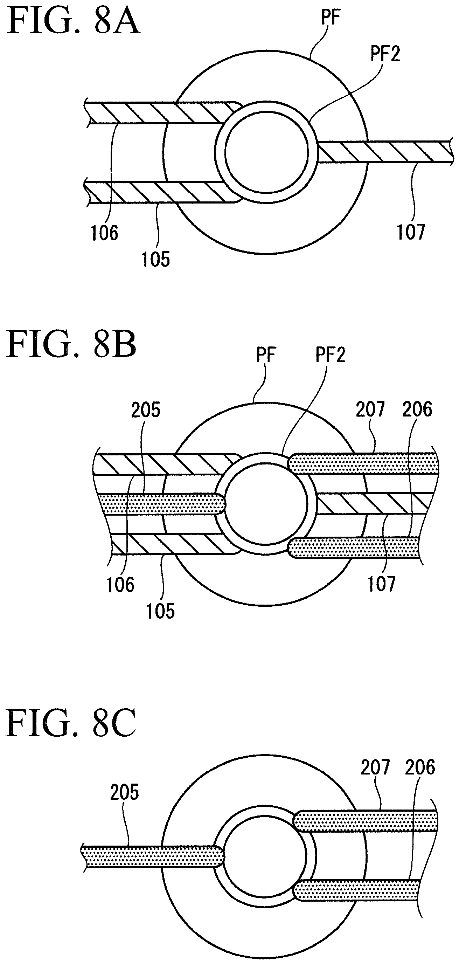

As an example thereof, grippers G suitable for eliminating areas that are not irradiated with the electron beam will be described with reference to FIGS. 7A and 7B and FIGS. 8A, 8B and 8C.

For the grippers G described here, as shown in FIGS. 7A and 7B and FIGS. 8A, 8B and 8C, for example, the gripper G constituting the gripper group 27C is referred to as a gripper 100, and the gripper G constituting the gripper group 29C is referred to as a gripper 200. That is, the gripper 100 is paired with the gripper 200, and in the sterilization unit 20, the preform PF gripped by the gripper 100 is transferred to the gripper 200, so that the grasp switching of the preform PF is performed.

As shown in FIG. 7A, the gripper 100 includes a pair of arms 101, 102 that grip the preform PF on the lower side of a neck ring PF3 and that are disposed with an interval. The arms 101, 102 are provided so as to be capable of performing opening-closing motion around rocking shafts 103, 104, respectively. As shown in FIG. 7A and FIG. 8A, the arm 101 includes two grip claws 105, 106 on the distal end side, and the arm 102 includes one grip claw 107 on the distal end side.

The gripper 100 is disposed such that the rocking shafts 103, 104 are on the lower side in the vertical direction and the grip claws 105, 106, 107 are on the upper side in the vertical direction, and grips the preform PF that is disposed between the arm 101 and the arm 102. As shown in FIG. 7A and FIG. 8A, with reference to the preform PF, the grip is performed from one side by the grip claws 105, 106, and is performed from the other side by the grip claw 107. As shown in FIG. 8A and FIG. 8B, the three grip claws 105, 106, 107 are disposed at an equal angle in the circumferential direction of the preform PF.

As shown in FIG. 7B, the gripper 200 includes a pair of arms 201, 202 that grip the preform PF on the upper side of the neck ring PF3 and that are disposed with an interval. The arms 201, 202 are bent near the center in the longitudinal direction, and are provided so as to preform opening-closing motion around rocking shafts 203, 204 that are provided at the bent portions respectively.

The arm 201 has a cam pin 208 fixed at a distal end part of an upper arm 201A located on the upper side than the rocking shaft 203, and includes a grip claw 205 at a distal end part of a lower arm 201B located on the lower side than the rocking shaft 203. Further, the arm 202 has a cam pin 209 fixed at a distal end part of an upper arm 202A located on the upper side than the rocking shaft 204, and includes two grip claws 206, 207 at a distal end part of a lower arm 202B located on the lower side than the rocking shaft 204. The gripper 200 is disposed such that the rocking shafts 203, 204 are on the upper side in the vertical direction and the grip claws 105, 106, 107 are on the lower side in the vertical direction, and grips the preform PF that is disposed between the arm 201 and the arm 202.

As shown in FIG. 7B, the gripper 200 includes a drive cam 211. The drive cam 211 rises and falls, and thereby, grips and releases the preform PF through the opening-closing operation of the arm 201 and the arm 202.

The drive cam 211 includes a cam plate 212 and a pair of cam grooves 214, 215 that are formed on the cam plate 212. The cam plate 212 performs rising-falling motion by an unillustrated actuator. The cam pin 208 for the upper arm 201A and the cam pin 209 for the lower arm 202B are inserted into the cam grooves 214, 215, respectively.

When the drive cam 211 is at the uppermost position (the left side in FIG. 7B), the preform PF is gripped by the grip claw 205 of the lower arm 201B and the grip claws 206, 207 of the lower arm 202B, and when the drive cam 211 is at the lowermost position (the right side in FIG. 7B), the grip of the preform PF is released.

As shown in FIG. 7A and FIG. 8B, with respect to the preform PF as the center, the grip is performed from one side by the grip claw 205, and is performed from the other side by the grip claws 206, 207. As shown in FIG. 8B and FIG. 8C, the three grip claws 205, 206, 207 are disposed at an equal angle in the circumferential direction of the preform PF.

With reference to the preform PF, the grip claw 105 of the gripper 100 and the two grip claws 205, 206 of the gripper 200 are disposed on the same side, and the grip claw 105 is disposed between the grip claws 205, 206. Further, the grip claws 106, 107 of the gripper 100 and the grip claw 207 of the gripper 200 are disposed on the same side, and the grip claw 207 is disposed between the grip claws 106, 106.

Accordingly, as shown in FIG. 8B, the grip claws 105, 106, 107 of the gripper 100 and the grip claws 205, 206, 207 of the gripper 200 can perform the grasp switching of the preform PF, without interference from each other. Moreover, the grip claws 105, 106, 107 of the gripper 100 and the grip claws 205, 206, 207 of the gripper 200 grip different positions on the preform PF, and therefore, do not grip identical positions before and after the grasp switching. Therefore, by using the gripper 100 and the gripper 200, it is possible to minimize sites that are not irradiated with the electron beam in a period before and after the grasp switching. Further, each of the gripper 100 and the gripper 200 adopts the three-point support structure, and therefore, it is possible to reduce hidden sites on the preforms PF. The three-point support structure is difficult to be applied to a gripper of the rotary conveyance device with use of a star wheel, but can be easily applied to the gripper that moves along the linear conveyance path.

The embodiment shows an example of the application to the blow-molding device 1 that molds the preform PF into the container B by blow molding, but the present invention can be applied to any object. For example, the present invention can be applied for sterilizing not the preform PF but the blow-molded container B, and the present invention can be applied to a sterilization object, other than the preform PF and the container B, that is conveyed while being gripped by a gripper.

Further, in the present invention, it is only necessary to irradiate an area in which the grasp switching by sterilization object grippers is performed, and the forward and rearward areas thereof, with the electron beam. That is, although the present embodiment shows an example in which the preform PF as the sterilization object is conveyed along the linear conveyance path, the present invention can be applied, for example, to a case in which the sterilization object is conveyed by a rotating body called a star wheel. Further, although the present embodiment adopts a batch process in which the sterilization process is performed for a predetermined number of preforms PF, the present invention can be applied, for example, to a case in which the sterilization process is consecutively performed for sterilization objects that are conveyed by the star wheel. The conveyance by the star wheel has an advantage in that it is possible to increase the number of sterilization objects that can be processed per unit time.

### Reference Signs List

1 blow-molding device
10 carry-in unit
11 rotating body
13 conveyance belt
15 conveyance means
20 sterilization unit
21 pre-irradiation chamber
23 irradiation chamber
24 electron beam generation device
24A upper irradiation unit
24B lower irradiation unit
124A emission surface
124B emission surface
25 post-irradiation chamber
27 first conveyance means
27A guide rail
27B running carriage
27C gripper group
29 second conveyance means
29A guide rail
29B running carriage
29C gripper group
31 third conveyance means
31A guide rail
31B running carriage
31C gripper group
40 molding unit
41, 43 molding die
42, 44 die element
45 fourth conveyance means
45A guide rail
45B running carriage
45C gripper group
60 control unit
100 gripper
101, 102 arm
103, 104 rocking shaft
105, 106, 107 grip claw
200 gripper
201, 202 arm
201A, 202A upper arm
201B, 202B lower arm
203, 204 rocking shaft
205, 206, 207 grip claw
208, 209 cam pin
211 drive cam
212 cam plate
214, 215 cam groove
B container
PF preform
PF1 opening part
PF2 bottom part
PF3 neck ring
EA electron beam irradiation area
EB electron beam
L1 first path
L2 second path

## Claims

1. An electron beam sterilization device comprising:
a first gripper (27C; 100) configured to receive a sterilization object (PF) and then convey the sterilization object in a conveyance direction to a grasp switching position (P23) while gripping the sterilization object;
a second gripper (29C; 200) configured to receive the sterilization object from the first gripper and then convey the sterilization object downstream while gripping the sterilization object; and
an electron beam irradiation unit (20) configured to irradiate the sterilization object that passes through an irradiation area (EA), with an electron beam (EB), the irradiation area containing the grasp switching position and forward and rearward positions of the grasp switching position,
**characterized in that** each of the first gripper (27C) and the second gripper (29C) is configured to move along a linear conveyance path while gripping a predetermined number of the sterilization objects without rotating the sterilization objects about the axis of the grippers (27C, 29C) .

2. The electron beam sterilization device according to claim 1, wherein
each of the first gripper (27C) and the second gripper (29C) are configured to grip the predetermined number of the sterilization objects (PF) that are arrayed on a line, and
one or both of the first gripper and the second gripper are configured to relatively come close to each other in a direction perpendicular to a direction in which a plurality of the sterilization objects are arrayed, so that grasp switching of the sterilization objects from the first gripper to the second gripper is performed at the grasp switching position (P23).

3. The electron beam sterilization device according to claim 1 or claim 2, wherein
the first gripper (27C) and the second gripper (29C) are configured to grip the sterilization object on different positions from each other respectively, and
the irradiation area (EA) for the electron beam (EB) in the electron beam irradiation unit (20) is put under a reduced-pressure atmosphere.

4. The electron beam sterilization device according to claim 3,
wherein
the electron beam irradiation unit (20) includes:
a pre-irradiation chamber (21) in which the first gripper (27C) receives the predetermined number of the sterilization objects (PF);
an irradiation chamber (23) in which the sterilization objects (PF) conveyed by the first gripper are irradiated with the electron beam (EB) while grasp switching of the sterilization objects to the second gripper (29C) is being performed;
a post-irradiation chamber (25) in which the sterilization objects irradiated with the electron beam in the irradiation chamber are conveyed by the second gripper; and
an electron beam generation device (20) configured to perform irradiation with the electron beam in the irradiation chamber, and
the irradiation chamber has a higher vacuum degree than the pre-irradiation chamber and the post-irradiation chamber.

5. The electron beam sterilization device according to any one of claims 1 to 4, wherein
the sterilization object (PF) is a container (B) including an opening part (PF1) and a storage space for holding a content, the storage space communicating with the opening part, or a preformed body (PF) for the container,
the sterilization object (PF) is configured to be gripped by the first gripper (27C) and the second gripper (29C), in an upright state in which the opening part is oriented upward in a vertical direction or in an inverted state that is upside-down, and
the electron beam irradiation unit (20) includes:
a first electron beam generation device (24A) configured to irradiate the sterilization object with the electron beam from an upper side to a lower side in a vertical direction; and
a second electron beam generation device (24B) configured to irradiate the sterilization object with the electron beam from the lower side to the upper side in the vertical direction.

6. The electron beam sterilization device according to claim 1, wherein each of the first gripper (27C) and the second gripper (29C) is configured to move along a linear conveyance path from upstream toward downstream while gripping the predetermined number of the sterilization objects that are arrayed on a line, one or both of the first gripper (27C) and the second gripper (29) are configured to relatively come close to each other in a direction perpendicular to a direction of the conveyance path, so that grasp switching of the sterilization objects from the first gripper to the second gripper is performed at the grasp switching position (P23).

7. An electron beam sterilization method comprising:
a step in which a first gripper (27C; 100) receives a sterilization object (PF) and then conveys the sterilization object in a conveyance direction to a grasp switching position (P23) while gripping the sterilization object;
a step in which a second gripper (29C; 200) receives the sterilization object from the first gripper and then conveys the sterilization object downstream while gripping the sterilization object; and
an electron beam irradiation step of irradiating the sterilization object that passes through an irradiation area (EA), with an electron beam (EB), the irradiation area containing the grasp switching position and forward and rearward positions of the grasp switching position,
wherein each of the first gripper (27C) and the second gripper (29C) moves along a linear conveyance path while gripping a predetermined number of the sterilization objects without rotating the sterilization objects about the axis of the grippers (27C, 29C).

8. The electron beam sterilization method according to claim 7, wherein
each of the first gripper (27C) and the second gripper (29C) grips the predetermined number of the sterilization objects that are arrayed on a line, and
one or both of the first gripper and the second gripper relatively come close to each other in a direction perpendicular to a direction in which a plurality of the sterilization objects are arrayed, so that grasp switching of the sterilization objects from the first gripper to the second gripper is performed at the grasp switching position (P23).

9. The electron beam sterilization method according to claim 7 or claim 8, wherein
the first gripper (27C) and the second gripper (29C) grip the sterilization object on different positions from each other, and
the irradiation area (EA) for the electron beam (EB) is put under a reduced-pressure atmosphere.

10. The electron beam sterilization method according to claim 9, comprising:
a pre-irradiation chamber (21) in which the first gripper (27C) receives the predetermined number of the sterilization objects (PF);
an irradiation chamber (23) in which the sterilization objects conveyed by the first gripper are irradiated with the electron beam (EB) while grasp switching of the sterilization objects to the second gripper (29C) is being performed;
a post-irradiation chamber (25) to which the sterilization objects irradiated with the electron beam (EB) in the irradiation chamber are conveyed by the second gripper; and
an electron beam generation device (20) configured to perform irradiation with the electron beam (EB) in the irradiation chamber,
wherein
the irradiation chamber (23) has a higher vacuum degree than the pre-irradiation chamber (21) and the post-irradiation chamber (25).

11. The electron beam sterilization method according to claim 10, wherein
the second gripper (29C) increases a pitch between the sterilization objects gripped, while the sterilization objects are being conveyed from the irradiation chamber (23) to the post-irradiation chamber (25) or after the sterilization objects are conveyed to the post-irradiation chamber (25).

12. The electron beam sterilization method according to any one of claims 7 to 11, wherein
the sterilization object is a container (B) including an opening part (PF1) and a storage space for holding a content, the storage space communicating with the opening part, or a preformed body (PF) for the container,
the sterilization object is gripped by the first gripper (27C) and the second gripper (29C), in an upright state in which the opening part is oriented upward in a vertical direction or in an inverted state that is upside-down, and
the sterilization object, with the electron beam (EB), is irradiated from an upper side to a lower side in a vertical direction, and is irradiated from the lower side to the upper side in the vertical direction.

13. The electron beam sterilization method according to claim 7, wherein
each of the first gripper (27C) and the second gripper (29C) moves along a linear conveyance path from upstream toward downstream while gripping the predetermined number of the sterilization objects that are arrayed on a line,
one or both of the first gripper and the second gripper relatively come close to each other in a direction perpendicular to a direction of the conveyance path, so that grasp switching of the sterilization objects from the first gripper to the second gripper is performed at the grasp switching position (P23).

## Patentansprüche

1. Elektronenstrahl-Sterilisationsvorrichtung umfassend:
einen ersten Greifer (27C; 100), der so konfiguriert ist, dass er ein Sterilisationsobjekt (PF) aufnimmt und dann das Sterilisationsobjekt in einer Förderrichtung zu einer Greifschaltposition (P23) befördert, während er das Sterilisationsobjekt greift;
einen zweiten Greifer (29C; 200), der so konfiguriert ist, dass er das Sterilisationsobjekt von dem ersten Greifer aufnimmt und dann das Sterilisationsobjekt stromabwärts befördert, während er das Sterilisationsobjekt greift; und
eine Elektronenstrahl-Bestrahlungseinheit (20), die so konfiguriert ist, dass sie das Sterilisationsobjekt, das einen Bestrahlungsbereich (EA) durchläuft, mit einem Elektronenstrahl (EB) bestrahlt, wobei der Bestrahlungsbereich die Greifschaltposition sowie vordere und hintere Positionen der Greifschaltposition enthält,
**dadurch gekennzeichnet, dass**
sowohl der erste Greifer (27C) als auch der zweite Greifer (29C) so konfiguriert sind, dass sie sich entlang einer linearen Förderbahn bewegen, während sie eine vorbestimmte Anzahl von Sterilisationsobjekten greifen, ohne die Sterilisationsobjekte um die Achse der Greifer (27C, 29C) zu drehen.

2. Elektronenstrahl-Sterilisationsvorrichtung nach Anspruch 1, wobei
sowohl der erste Greifer (27C) als auch der zweite Greifer (29C) so konfiguriert sind, dass sie die vorbestimmte Anzahl von Sterilisationsobjekten (PF) greifen, die auf einer Linie angeordnet sind, und
einer oder beide des ersten Greifers und des zweiten Greifers, so konfiguriert sind, dass sie sich in einer Richtung senkrecht zu einer Richtung, in der eine Vielzahl der Sterilisationsobjekte angeordnet sind, relativ nahe kommen, sodass das Umschalten des Greifens der Sterilisationsobjekte von dem ersten Greifer auf den zweiten Greifer an der Greifschaltposition (P23) durchgeführt wird.

3. Elektronenstrahl-Sterilisationsvorrichtung nach Anspruch 1 oder Anspruch 2, wobei
der erste Greifer (27C) und der zweite Greifer (29C) so konfiguriert sind, dass sie das Sterilisationsobjekt an jeweils unterschiedlichen Positionen greifen, und
der Bestrahlungsbereich (EA) für den Elektronenstrahl (EB) in der Elektronenstrahl-Bestrahlungseinheit (20) unter eine Atmosphäre mit reduziertem Druck gesetzt wird.

4. Elektronenstrahl-Sterilisationsvorrichtung nach Anspruch 3, wobei die Elektronenstrahl-Bestrahlungseinheit (20) Folgendes beinhaltet:
eine Vorbestrahlungskammer (21), in der der erste Greifer (27C) die vorbestimmte Anzahl der Sterilisationsobjekte (PF) aufnimmt;
eine Bestrahlungskammer (23), in der die vom ersten Greifer beförderten Sterilisationsobjekte (PF) mit dem Elektronenstrahl (EB) bestrahlt werden, während das Umschalten des Greifens der Sterilisationsobjekte auf den zweiten Greifer (29C) durchgeführt wird;
eine Nachbestrahlungskammer (25), in die die mit dem Elektronenstrahl bestrahlten Sterilisationsobjekte in der Bestrahlungskammer durch den zweiten Greifer befördert werden; und
eine Elektronenstrahl-Erzeugungsvorrichtung (20), die so konfiguriert ist, dass sie eine Bestrahlung mit dem Elektronenstrahl in der Bestrahlungskammer durchführt, und
die Bestrahlungskammer einen höheren Vakuumgrad aufweist als die Vorbestrahlungskammer und die Nachbestrahlungskammer.

5. Elektronenstrahl-Sterilisationsvorrichtung nach einem der Ansprüche 1 bis 4, wobei
das Sterilisationsobjekt (PF) ein Behälter (B) ist, der einen Öffnungsteil (PF1) und einen Stauraum zur Aufnahme eines Inhalts enthält, wobei der Stauraum mit dem Öffnungsteil in Verbindung steht, oder ein vorgeformter Körper (PF) für den Behälter,
das Sterilisationsobjekt (PF) so konfiguriert ist, dass es von dem ersten Greifer (27C) und dem zweiten Greifer (29C) in einem aufrechten Zustand, in dem der Öffnungsteil in einer vertikalen Richtung nach oben gerichtet ist, oder in einem umgekehrten Zustand, der auf dem Kopf steht, gegriffen wird, und
die Elektronenstrahl-Bestrahlungseinheit (20) Folgendes beinhaltet:
eine erste Elektronenstrahl-Erzeugungsvorrichtung (24A), die so konfiguriert ist, dass sie das Sterilisationsobjekt mit dem Elektronenstrahl von einer Oberseite zu einer Unterseite in einer vertikalen Richtung bestrahlt; und
eine zweite Elektronenstrahl-Erzeugungsvorrichtung (24B), die so konfiguriert ist, dass sie das Sterilisationsobjekt mit dem Elektronenstrahl von der unteren Seite zur oberen Seite in vertikaler Richtung bestrahlt.

6. Elektronenstrahl-Sterilisationsvorrichtung nach Anspruch 1, wobei sowohl der erste Greifer (27C) als auch der zweite Greifer (29C) so konfiguriert sind, dass sie sich entlang einer linearen Förderbahn von stromaufwärts nach stromabwärts bewegen, während sie die vorbestimmte Anzahl von Sterilisationsobjekten greifen, die in einer Reihe angeordnet sind, wobei einer oder beide des ersten Greifers (27C) und des zweiten Greifers (29), so konfiguriert sind, dass sie sich in einer Richtung senkrecht zu einer Richtung der Förderbahn relativ nahe kommen, sodass das Umschalten des Greifens der Sterilisationsobjekte von dem ersten Greifer auf den zweiten Greifer an der Greifschaltposition (P23) durchgeführt wird.

7. Elektronenstrahl-Sterilisationsverfahren, umfassend:
einen Schritt, bei dem ein erster Greifer (27C; 100) ein Sterilisationsobjekt (PF) aufnimmt und dann das Sterilisationsobjekt in einer Förderrichtung zu einer Greifschaltposition (P23) befördert, während er das Sterilisationsobjekt greift;
einen Schritt, in dem ein zweiter Greifer (29C; 200) das Sterilisationsobjekt vom ersten Greifer empfängt und dann das Sterilisationsobjekt stromabwärts befördert, während er das Sterilisationsobjekt greift; und
einen Elektronenstrahl-Bestrahlungsschritt, bei dem das Sterilisationsobjekt, das einen Bestrahlungsbereich (EA) durchläuft, mit einem Elektronenstrahl (EB) bestrahlt wird, wobei der Bestrahlungsbereich die Greifschaltposition sowie vordere und hintere Positionen der Greifschaltposition enthält,
wobei der erste Greifer (27C) und der zweite Greifer (29C) sich jeweils entlang einer linearen Förderbahn bewegen, während sie eine vorbestimmte Anzahl der Sterilisationsobjekte greifen, ohne die Sterilisationsobjekte um die Achse der Greifer (27C, 29C) zu drehen.

8. Elektronenstrahl-Sterilisationsverfahren nach Anspruch 7, wobei
sowohl der erste Greifer (27C) als auch der zweite Greifer (29C) die vorbestimmte Anzahl der Sterilisationsobjekte, die auf einer Linie angeordnet sind, greift, und
einer oder beide des ersten Greifers und des zweiten Greifers in einer Richtung senkrecht zu einer Richtung, in der eine Vielzahl der Sterilisationsobjekte angeordnet sind, relativ nahe beieinander liegen, sodass das Umschalten des Greifens der Sterilisationsobjekte von dem ersten Greifer auf den zweiten Greifer an der Greifschaltposition (P23) durchgeführt wird.

9. Elektronenstrahl-Sterilisationsverfahren nach Anspruch 7 oder Anspruch 8, wobei
der erste Greifer (27C) und der zweite Greifer (29C) das Sterilisationsobjekt an unterschiedlichen Positionen greifen, und
der Bestrahlungsbereich (EA) für den Elektronenstrahl (EB) unter eine Atmosphäre mit reduziertem Druck gesetzt wird.

10. Elektronenstrahl-Sterilisationsverfahren nach Anspruch 9, umfassend:
eine Vorbestrahlungskammer (21), in der der erste Greifer (27C) die vorbestimmte Anzahl der Sterilisationsobjekte (PF) aufnimmt;
eine Bestrahlungskammer (23), in der die vom ersten Greifer beförderten Sterilisationsobjekte mit dem Elektronenstrahl (EB) bestrahlt werden, während das Umschalten des Greifens der Sterilisationsobjekte auf den zweiten Greifer (29C) durchgeführt wird;
eine Nachbestrahlungskammer (25), in die die mit dem Elektronenstrahl (EB) bestrahlten Sterilisationsobjekte in der Bestrahlungskammer durch den zweiten Greifer befördert werden; und
eine Elektronenstrahl-Erzeugungsvorrichtung (20), die so konfiguriert ist, dass sie eine Bestrahlung mit dem Elektronenstrahl (EB) in der Bestrahlungskammer durchführt,
wobei
wobei die Bestrahlungskammer (23) einen höheren Vakuumgrad aufweist als die Vorbestrahlungskammer (21) und die Nachbestrahlungskammer (25).

11. Elektronenstrahl-Sterilisationsverfahren nach Anspruch 10, wobei
der zweite Greifer (29C) einen Abstand zwischen den gegriffenen Sterilisationsobjekten vergrößert, während die Sterilisationsobjekte von der Bestrahlungskammer (23) zur Nachbestrahlungskammer (25) befördert werden oder nachdem die Sterilisationsobjekte zur Nachbestrahlungskammer (25) befördert wurden.

12. Elektronenstrahl-Sterilisationsverfahren nach einem der Ansprüche 7 bis 11, wobei
das Sterilisationsobjekt ein Behälter (B) ist, der einen Öffnungsteil (PF1) und einen Stauraum zur Aufnahme eines Inhalts beinhaltet, wobei der Stauraum mit dem Öffnungsteil in Verbindung steht, oder ein vorgeformter Körper (PF) für den Behälter,
wobei das Sterilisationsobjekt von dem ersten Greifer (27C) und dem zweiten Greifer (29C) in einem aufrechten Zustand, in dem der Öffnungsteil in vertikaler Richtung nach oben gerichtet ist, oder in einem umgekehrten Zustand, der auf dem Kopf steht, gegriffen wird, und
das Sterilisationsobjekt mit dem Elektronenstrahl (EB) von einer oberen Seite zu einer unteren Seite in vertikaler Richtung bestrahlt wird und von der unteren Seite zur oberen Seite in vertikaler Richtung bestrahlt wird.

13. Elektronenstrahl-Sterilisationsverfahren nach Anspruch 7, wobei
wobei sowohl der erste Greifer (27C) als auch der zweite Greifer (29C) sich entlang einer linearen Förderbahn von stromaufwärts nach stromabwärts bewegt, während sie die vorbestimmte Anzahl von Sterilisationsobjekten greifen, die auf einer Linie angeordnet sind,
einer oder beide des ersten Greifers und des zweiten Greifers in einer Richtung senkrecht zu einer Richtung der Förderbahn relativ nahe beieinander liegen, sodass das Umschalten des Greifens der Sterilisationsobjekte von dem ersten Greifer auf den zweiten Greifer in der Greifschaltposition (P23) durchgeführt wird.

## Revendications

1. Dispositif de stérilisation par faisceau d'électrons comprenant :
une première pince de préhension (27C ; 100) configurée pour recevoir un objet de stérilisation (PF) et ensuite transporter l'objet de stérilisation dans une direction de transport vers une position de changement de préhension (P23) tout en enserrant l'objet de stérilisation ;
une seconde pince de préhension (29C ; 200) configurée pour recevoir l'objet de stérilisation à partir de la première pince de préhension et ensuite transporter l'objet de stérilisation vers l'aval tout en enserrant l'objet de stérilisation ; et
une unité d'irradiation par faisceau d'électrons (20) configurée pour irradier l'objet de stérilisation qui traverse une zone d'irradiation (EA), avec un faisceau d'électrons (EB), la zone d'irradiation contenant la position de changement de préhension et les positions avant et arrière de la position de changement de préhension,
**caractérisé en ce que**
chacune parmi la première pince de préhension (27C) et la seconde pince de préhension (29C) est configurée pour se déplacer le long d'un trajet de transport linéaire tout en enserrant un nombre prédéterminé d'objets de stérilisation sans faire tourner les objets de stérilisation autour de l'axe des pinces de préhension (27C, 29C).

2. Dispositif de stérilisation par faisceau d'électrons selon la revendication 1, dans lequel
chacune parmi la première pince de préhension (27C) et la seconde pince de préhension (29C) est configurée pour enserrer le nombre prédéterminé d'objets de stérilisation (PF) qui sont disposés sur une ligne, et
une ou les deux parmi la première pince de préhension et la seconde pince de préhension sont configurées pour se rapprocher de manière relative l'une de l'autre dans une direction perpendiculaire à une direction dans laquelle une pluralité d'objets de stérilisation sont disposés, de sorte que le changement de préhension des objets de stérilisation depuis la première pince de préhension vers la seconde pince de préhension s'effectue à la position de changement de préhension (P23).

3. Dispositif de stérilisation par faisceau d'électrons selon la revendication 1 ou la revendication 2, dans lequel
la première pince de préhension (27C) et la seconde pince de préhension (29C) sont configurées pour enserrer l'objet de stérilisation sur des positions différentes les unes des autres respectivement, et
la zone d'irradiation (EA) pour le faisceau d'électrons (EB) dans l'unité d'irradiation par faisceau d'électrons (20) est placée sous une atmosphère à pression réduite.

4. Dispositif de stérilisation par faisceau d'électrons selon la revendication 3, dans lequel
l'unité d'irradiation par faisceau d'électrons (20) inclut :
une chambre de pré-irradiation (21) dans laquelle la première pince de préhension (27C) reçoit le nombre prédéterminé d'objets de stérilisation (PF) ;
une chambre d'irradiation (23) dans laquelle les objets de stérilisation (PF) transportés par la première pince de préhension sont irradiés avec le faisceau d'électrons (EB) tandis qu'un changement de préhension des objets de stérilisation vers la seconde pince de préhension (29C) est effectué ;
une chambre de post-irradiation (25) dans laquelle les objets de stérilisation irradiés par le faisceau d'électrons dans la chambre d'irradiation sont transportés par la seconde pince de préhension ; et
un dispositif de génération de faisceau d'électrons (20) configuré pour effectuer une irradiation avec le faisceau d'électrons dans la chambre d'irradiation, et
la chambre d'irradiation présente un degré de vide plus élevé que la chambre de pré-irradiation et la chambre de post-irradiation.

5. Dispositif de stérilisation par faisceau d'électrons selon l'une quelconque des revendications 1 à 4, dans lequel
l'objet de stérilisation (PF) est un récipient (B) incluant une partie ouverture (PF1) et un espace de stockage pour contenir un contenu, l'espace de stockage communiquant avec la partie ouverture, ou un corps préformé (PF) pour le récipient,
l'objet de stérilisation (PF) est configuré pour être enserré par la première pince de préhension (27C) et la seconde pince de préhension (29C), dans un état vertical dans lequel la partie ouverture est orientée vers le haut dans une direction verticale ou dans un état inversé qui est à l'envers, et
l'unité d'irradiation par faisceau d'électrons (20) inclut :
un premier dispositif de génération de faisceau d'électrons (24A) configuré pour irradier l'objet de stérilisation avec le faisceau d'électrons d'un côté supérieur vers un côté inférieur dans une direction verticale ; et
un second dispositif de génération de faisceau d'électrons (24B) configuré pour irradier l'objet de stérilisation avec le faisceau d'électrons à partir du côté inférieur vers le côté supérieur dans la direction verticale.

6. Dispositif de stérilisation par faisceau d'électrons selon la revendication 1, dans lequel chacune parmi la première pince de préhension (27C) et la seconde pince de préhension (29C) est configurée pour se déplacer le long d'un trajet de transport linéaire de l'amont vers l'aval tout en enserrant le nombre prédéterminé d'objets de stérilisation qui sont disposés sur une ligne, l'une ou les deux parmi la première pince de préhension (27C) et la seconde pince de préhension (29) sont configurées pour se rapprocher de manière relative l'une de l'autre dans une direction perpendiculaire à une direction du trajet de transport, de sorte que le changement de préhension des objets de stérilisation depuis la première pince de préhension vers le seconde pince de préhension s'effectue à la position de changement de préhension (P23).

7. Procédé de stérilisation par faisceau d'électrons comprenant :
une étape dans laquelle une première pince de préhension (27C ; 100) reçoit un objet de stérilisation (PF) et ensuite transporte l'objet de stérilisation dans une direction de transport vers une position de changement de préhension (P23) tout en enserrant l'objet de stérilisation ;
une étape dans laquelle une seconde pince de préhension (29C ; 200) reçoit l'objet de stérilisation à partir de la première pince de préhension et ensuite transporte l'objet de stérilisation vers l'aval tout en enserrant l'objet de stérilisation ; et
une étape d'irradiation par faisceau d'électrons consistant à irradier l'objet de stérilisation qui traverse une zone d'irradiation (EA), avec un faisceau d'électrons (EB), la zone d'irradiation contenant la position de changement de préhension et les positions avant et arrière de la position de changement de préhension,
dans lequel chacune parmi la première pince de préhension (27C) et la seconde pince de préhension (29C) se déplace le long d'un trajet de transport linéaire tout en enserrant un nombre prédéterminé d'objets de stérilisation sans faire tourner les objets de stérilisation autour de l'axe des pinces de préhension (27C, 29C).

8. Procédé de stérilisation par faisceau d'électrons selon la revendication 7, dans lequel
chacune parmi la première pince de préhension (27C) et la seconde pince de préhension (29C) enserre le nombre prédéterminé d'objets de stérilisation qui sont disposés sur une ligne, et
une ou les deux parmi la première pince de préhension et la seconde pince de préhension se rapprochent de manière relative l'une de l'autre dans une direction perpendiculaire à une direction dans laquelle une pluralité d'objets de stérilisation sont disposés, de sorte que le changement de préhension des objets de stérilisation depuis la première pince de préhension vers la seconde pince de préhension s'effectue à la position de changement de préhension (P23).

9. Procédé de stérilisation par faisceau d'électrons selon la revendication 7 ou la revendication 8, dans lequel
la première pince de préhension (27C) et la seconde pince de préhension (29C) enserrent l'objet de stérilisation sur des positions différentes les unes des autres, et
la zone d'irradiation (EA) pour le faisceau d'électrons (EB) est placée sous une atmosphère à pression réduite.

10. Procédé de stérilisation par faisceau d'électrons selon la revendication 9, comprenant :
une chambre de pré-irradiation (21) dans laquelle la première pince de préhension (27C) reçoit le nombre prédéterminé d'objets de stérilisation (PF) ;
une chambre d'irradiation (23) dans laquelle les objets de stérilisation transportés par la première pince de préhension sont irradiés avec le faisceau d'électrons (EB) tandis qu'un changement de préhension des objets de stérilisation vers la seconde pince de préhension (29C) est effectué ;
une chambre de post-irradiation (25) vers laquelle les objets de stérilisation irradiés par le faisceau d'électrons (EB) dans la chambre d'irradiation sont transportés par la seconde pince de préhension ; et
un dispositif de génération de faisceau d'électrons (20) configuré pour effectuer une irradiation avec le faisceau d'électrons (EB) dans la chambre d'irradiation,
dans lequel
la chambre d'irradiation (23) présente un degré de vide plus élevé que la chambre de pré-irradiation (21) et la chambre de post-irradiation (25).

11. Procédé de stérilisation par faisceau d'électrons selon la revendication 10, dans lequel
la seconde pince de préhension (29C) augmente un pas entre les objets de stérilisation enserrés, tandis que les objets de stérilisation sont transportés à partir de la chambre d'irradiation (23) vers la chambre de post-irradiation (25) ou après que les objets de stérilisation sont transportés vers la chambre de post-irradiation (25).

12. Procédé de stérilisation par faisceau d'électrons selon l'une quelconque des revendications 7 à 11, dans lequel
l'objet de stérilisation est un récipient (B) incluant une partie ouverture (PF1) et un espace de stockage pour contenir un contenu, l'espace de stockage communiquant avec la partie ouverture, ou un corps préformé (PF) pour le récipient,
l'objet de stérilisation est enserré par la première pince de préhension (27C) et la seconde pince de préhension (29C), dans un état vertical dans lequel la partie ouverture est orientée vers le haut dans une direction verticale ou dans un état inversé qui est à l'envers, et
l'objet de stérilisation, avec le faisceau d'électrons (EB), est irradié à partir d'un côté supérieur vers un côté inférieur dans une direction verticale, et est irradié à partir du côté inférieur vers le côté supérieur dans la direction verticale.

13. Procédé de stérilisation par faisceau d'électrons selon la revendication 7, dans lequel
chacune parmi la première pince de préhension (27C) et la seconde pince de préhension (29C) se déplace le long d'un trajet de transport linéaire de l'amont vers l'aval tout en enserrant le nombre prédéterminé d'objets de stérilisation qui sont disposés sur une ligne,
une ou les deux parmi la première pince de préhension et la seconde pince de préhension se rapprochent de manière relative l'une de l'autre dans une direction perpendiculaire à une direction du trajet de transport, de sorte que le changement de préhension des objets de stérilisation depuis la première pince de préhension vers la seconde pince de préhension s'effectue à la position de changement de préhension (P23).
